(19) Europäisches Patentamt

European Patent Office

Office européen des brevets

(11) **EP 0 976 834 B1**

(12) **EUROPEAN PATENT SPECIFICATION**

(45) Date of publication and mention
of the grant of the patent:
**10.03.2004 Bulletin 2004/11**

(51) Int Cl.[7]: **C12Q 1/32**, C12N 15/53,
C12N 1/21

(21) Application number: **99114742.2**

(22) Date of filing: **28.07.1999**

(54) **Liquid reagent for calcium assay**

Flüssiges Reagens zur Bestimmung von Kalzium

Réactif liquide pour l'essai de calcium

(84) Designated Contracting States:
**DE FR GB IT**

(30) Priority: **30.07.1998 JP 22856598**
**25.05.1999 JP 14517399**

(43) Date of publication of application:
**02.02.2000 Bulletin 2000/05**

(73) Proprietor: **ORIENTAL YEAST CO., LTD.**
**Tokyo (JP)**

(72) Inventors:
• **Matsukawa, Hirokazu**
**Moriguchi-shi, Osaka-fu (JP)**
• **Oka, Osamu**
**Kyoto-shi, Kyoto-fu (JP)**
• **Fujita, Tuyosi**
**Ikeda-shi, Osaka-fu (JP)**
• **Sakakibara, Hitoshi**
**Mukou-shi, Kyoto-fu (JP)**
• **Sugiyama, Tatsuo**
**Nissin-shi, Aichi-ken (JP)**

(74) Representative:
**Gille Hrabal Struck Neidlein Prop Roos**
**Patentanwälte**
**Brucknerstrasse 20**
**40593 Düsseldorf (DE)**

(56) References cited:
**EP-A- 0 035 831        EP-A- 0 555 046**
**WO-A-87/06264**

• **CHEMICAL ABSTRACTS, vol. 123, no. 21, 20
November 1995 (1995-11-20) Columbus, Ohio,
US; abstract no. 277526, H. SAKAKIBARA ET
AL.: "Isolation and characterization of a cDNA
that encodes maize glutamate dehydrogenase"
page 310; column 1; XP002135439 & PLANT
CELL PHYSIOLOGY, vol. 36, no. 5, 1995, pages
789-797,**
• **M. P. PURNELL ET AL.: "Cloning and
characterisation of a glutamate dehydrogenase
cDNA from tomato (Lycopersicon esculentum
L.)" GENE: AN INTERNATIONAL JOURNAL ON
GENES AND GENOMES., vol. 186, 1997, pages
249-254, XP004056137 ELSEVIER SCIENCE
PUBLISHERS, BARKING., GB ISSN: 0378-1119**
• **Loulakakis CA. AND Roubelakis-Angelakis KA.:
"Intracellular Localization and Properties of
NADH-Glutamate Dehydrogenase from Vits
vinifer L.....", J. Exp. Bot. (1990), Vol. 41, No. 231,
pages 1223-1230**
• **SAKAKIBARA H. ET AL: 'Isolation and
Characterization of a cDNA that Encodes Maize
Glutamate Dehydrogenase' PLANT CELL
PHYSIOLOGY vol. 36, no. 5, 1995, pages 789 -
797**

SEQUENCE LISTING

<110> Oriental Yeast Co., LTD.
<120> Liquid Reagent for Calcium Determination
(130) 6161
<141> 1999-5-25
<160> 1
<210> 1
<211> 411
<212> PRT
<213> Maize
<400> 1

```
Met Asn Ala Leu Ala Ala Thr Ser Arg Asn Phe Lys Gln Ala Ala
1               5                   10                  15

Lys Leu Leu Gly Leu Asp Ser Lys Leu Glu Lys Ser Leu Leu Ile
                20                  25                  30

Pro Phe Arg Glu Ile Lys Val Glu Cys Thr Ile Pro Lys Asp Asp
                35                  40                  45

Gly Thr Leu Ala Ser Tyr Val Gly Phe Arg Val Gln His Asp Asn
                50                  55                  60

Ala Arg Gly Pro Met Lys Gly Gly Ile Arg Tyr His His Glu Val
                65                  70                  75

Asp Pro Asp Glu Val Asn Ala Leu Ala Gln Leu Met Thr Trp Lys
                80                  85                  90

Thr Ala Val Ala Asn Ile Pro Tyr Gly Gly Ala Lys Gly Gly Ile
                95                  100                 105

Gly Cys Ser Pro Gly Asp Leu Ser Ile Ser Glu Leu Glu Arg Leu
```

```
              110               115               120
Thr Arg Val Phe Thr Gln Lys Ile His Asp Leu Ile Gly Ile His
              125               130               135
Thr Asp Val Pro Ala Pro Asp Met Gly Thr Asn Ser Gln Thr Met
              140               145               150
Ala Trp Ile Leu Asp Glu Tyr Ser Lys Phe His Gly Tyr Ser Pro
              155               160               165
Ala Val Val Thr Gly Lys Pro Val Asp Leu Gly Gly Ser Leu Gly
              170               175               180
Arg Asp Ala Ala Thr Gly Arg Gly Val Leu Phe Ala Thr Glu Ala
              185               190               195
Leu Leu Ala Glu His Gly Lys Gly Ile Ala Gly Gln Arg Phe Val
              200               205               210
Ile Gln Gly Phe Gly Asn Val Gly Ser Trp Ala Ala Gln Leu Ile
              215               220               225
Ser Glu Ala Gly Gly Lys Val Ile Ala Ile Ser Asp Val Thr Gly
              230               235               240
Ala Val Lys Asn Val Asp Gly Leu Asp Ile Ala Gln Leu Val Lys
              245               250               255
His Ser Ala Glu Asn Lys Gly Ile Lys Gly Phe Lys Gly Gly Asp
              260               265               270
Ala Ile Ala Pro Asp Ser Leu Leu Thr Glu Glu Cys Asp Val Leu
              275               280               285
Ile Pro Ala Ala Leu Gly Gly Val Ile Asn Lys Asp Asn Ala Asn
              290               295               300
Asp Ile Lys Ala Lys Tyr Ile Ile Glu Ala Ala Asn His Pro Thr
              305               310               315
```

```
Asp Pro Glu Ala Asp Glu Ile Leu Ser Lys Lys Gly Val Leu Ile
            320               325               330

Leu Pro Asp Ile Leu Ala Asn Ser Gly Gly Val Thr Val Ser Tyr
            335               340               345

Phe Glu Trp Val Gln Asn Ile Gln Gly Phe Met Trp Asp Glu Glu
            350               355               360

Lys Val Asn Ala Glu Leu Arg Thr Tyr Ile Thr Arg Ala Phe Gly
            365               370               375

Asn Val Lys Gln Met Cys Arg Ser His Ser Cys Asp Leu Arg Met
            380               385               390

Gly Ala Phe Thr Leu Gly Val Asn Arg Val Ala Arg Ala Thr Val
            395               400               405

Leu Arg Gly Trp Glu Ala
            410
```

**Description**

Detailed Description of the Invention

Technical Field to which the Invention Belongs

The present invention relates to a liquid reagent for assaying calcium in body fluid samples. Characteristically, the inventive reagent contains a specific calcium-dependent glutamate dehydrogenase and a chelating agent and is in a liquid form, and consists of a kit containing two separate containers.

Additionally, the invention relates to a method for assaying calcium in body fluid samples by using the assay reagent.

Prior Art

Calcium ion plays a significant role in biological organisms. In biological organisms, 99 % of calcium is locally present in bone and teeth, but calcium is continuously absorbed in bone or excreted therefrom at 700 mg/day. Thus, calcium is also present in body fluids and cells at a lesser amount than the amount in bone. The calcium in body fluids and cells is responsible for significant functions as second messenger in biological actions, including nerve transmission function, muscle contraction function, and hormonal actions, in addition to blood coagulation. Thus, the calcium level in body fluids, particularly in blood, should be retained constantly in a strict manner. It has been known that blood calcium level is retained constantly by vitamin D, parathyroid hormone and calcitonin; in normal individuals, for example, calcium is present at 9 to 11 mg/dl in blood and the intra-day variation of the calcium level should be strictly retained within ± 3 % at most. The calcium level varies in response to diseased conditions. Hypercalcemia occurs in diseases such as myxedema, malignant tumor sarcoidosis, hyperproteinemia, and hyperthyroidism; hypocalcemia emerges in diseases such as hypoparathyroidism, osteomalacia, renal rickets, uremia, hypoproteinemia, and bone metastasis of malignant tumor. Even a slight change of the blood calcium level in normal individuals simply indicates that the change is due to a disease. Thus, the blood calcium assay is a very important test item for laboratory tests.

Journal of Exp. Biol. Vol. 41, No. 231, pp. 1223 ff. discloses a Ca-dependent GLDH from grapevine and physicochemical properties thereof.

EP-A-0 555 046 discloses a method of determining Ca by using a transglutaminase capable of being activated with calcium.

As has been described above, the development of an excellent method for accurately assaying calcium in a simple manner has been expected, while many of assay reagent compositions in freeze-dried powder for laboratory tests are likely to be replaced with assay reagent compositions in solutions. This is because laborious works to dissolve powdery assay reagent compositions in liquids just prior to every use can thereby be skipped at clinical test practice to reduce the burdensome works on the site. The development of a simple liquid reagent for calcium assay is also demanded. From the standpoint of the stability of calcium assay reagents against problematic accidental temperature change under storage, in particular, a'calcium assay reagent suitable for long-term storage is demanded.

Problems that the Invention is to Solve

The invention has been achieved so as to satisfy such demands in the industry. It is an object of the invention to develop a novel calcium assay liquid reagent storable for a long term despite the liquid form.

Brief Description of the Drawings

Fig. 1 depicts the mechanism of GLDH enzyme reaction;
Fig. 2 depicts the mechanism of calcium assay by GLDH;
Fig. 3 depicts the responsiveness of the concentration-dependent activity within the serum calcium concentration range; and
Fig. 4 is a graph of a calcium standard curve with NTA as the adjusting factor.

Means for Solving the Problems

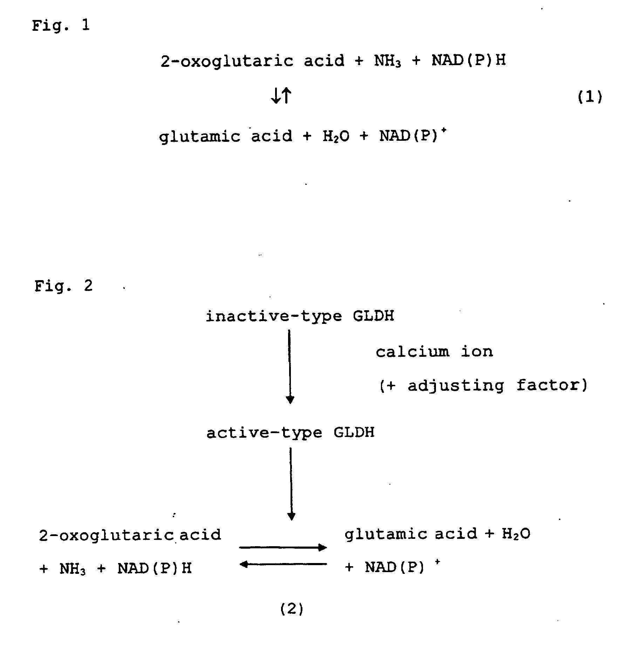

The aforementioned object is attained by the invention. The inventors have made investigations from the standpoint of a wide variety of fields. The inventors have concentrated their attention to glutamate dehydrogenase (referred to as GLDH hereinafter). Generally, GLDH is an enzyme catalyzing the reaction represented by the formula 1 in Fig. 1, including for example enzymes represented by Enzyme Nos. EC 1.4.1.2., EC 1.4.1.3. and EC 1.4.1.4.

Among them, the enzyme of the Enzyme No. EC 1.4.1.2. specific to $NAD^+$ (nicotinamide adenine dinucleotide; at oxidized state) is distributed in animal tissues, plants and bacteria. The enzyme of the Enzyme No. EC 1.4.1.3. is reactive with both of $NAD^+$ and $NADP^+$ (nicotinamide adenine dinucleotide phosphate; at oxidized state) and is locally present in animal liver and renal mitochondria. The enzyme of the Enzyme No. EC 1.4.1.4. present in yeast and bacteria is specific to $NADP^+$.

The inventors have further promoted investigations about GLDH. The inventors have focused attention particularly to a GLDH species with no activity exertion unless calcium ion is present (namely, calcium-dependent GLDH) among these GLDH species. Then, the inventors have attempted to contact the calcium-dependent enzyme (GLDH) to the calcium ion in a sample. Consequently, the inventors have first found that the GLDH enzyme activity varies in the presence of a substrate, depending on the calcium ion concentration in a sample and that the assay of the activity enables the assay of calcium ion.' After further investigations, consequently, the inventors have newly found that the calcium-dependent GLDH is stable in a liquid phase against temperature change under storage and can therefore retain a high enzyme activity in a solution for a long term. The invention has been achieved when the inventors have selected the calcium-dependent GLDH for a calcium assay system (in a liquid phase) for biological materials. Based on these new and useful findings, the invention has been achieved. In accordance with the invention, characteristically, the calcium-dependent GLDH is used for a liquid reagent for assaying calcium derived from a biological material in a sample.

In accordance with the assay method of calcium in body fluid samples, the activity of the GLDH is utilized.

More specifically, inactive-type GLDH is converted to active-type GLDH by utilizing calcium ion present in samples such as blood, serum, plasma and pancreatic juice. Then, ammonia, 2-oxoglutaric acid, NAD(P)H and the active-type GLDH are allowed to react together. By subsequently assaying for example the rate of NAD(P)H decrease on the basis of the absorbance around 340 nm, the amount of calcium derived from the biological material is assayed. Through the reaction represented by the formula 2 in Fig. 2, for example, the amount of calcium can be determined.

(Calcium-dependent GLDH)

In accordance with the invention, calcium-dependent GLDH is utilized as shown in the aforementioned reaction. The calcium-dependent GLDH defined in claim 1 is utilized which never exerts the activity in the absence of calcium ion. It is a Calcium-dependent GLDH derived from maize. The specific maize-derived GLDH can retain the enzyme activity even after thermal treatment under conditions of 70 °C and pH 7.0 for 20 minutes. A liquid reagent for calcium assay can be provided, by using therein an enzyme stable in a solution state under storage.

The amino acid sequence of the calcium-dependent GLDH derived from maize and the nucleotide sequence of the gene encoding the same are described in Sakakibara, H. et al., "Isolation and characterization of a cDNA that encodes maize glutamate dehydrogenase", Plant Cell Physiol. 36, 5: 789-797, 1995.

The amino acid sequence is shown as SEQ ID No. 1

The inventive calcium-dependent GLDH can be extracted and purified from maize by known methods including for example the method for the maize-derived enzyme as described in Plant Cell Physiol. 36, 5: 789-797, 1995. The calcium-dependent GLDH used according to the invention is satisfactorily a synthetic GLDH or a recombinant GLDH recovered by genetic engineering. A person skilled in the art can readily recover the calcium-dependent GLDH, according to the disclosure of the present Specification.

As described above, the amino acid sequence of the calcium-dependent GLDH and the nucleotide sequence of the gene encoding the calcium-dependent GLDH have been elucidated by the inventors; and therefore, such GLDH can be not only extracted from natural products such as maize but also synthetically prepared or industrially prepared in a genetic engineering manner by inserting a DNA fragment of the GLDH gene in a known expression vector such as commercially available expression vectors to prepare a plasmid, transforming a host such as Escherichia coli by using the resulting plasmid, and culturing the transformant to recover the objective GLDH.

(Liquid reagent containing calcium-dependent GLDH)

The inventive liquid reagent characteristically contains the calcium-dependent GLDH in an aqueous medium as a first reagent. The calcium-dependent GLDH is contained at a concentration of 0.1 U/ml to 50 U/ml, preferably 0.5 U/ml to 10 U/ml. As to the GLDH unit, herein, one unit of the enzyme is defined as the amount thereof to generate 1 μmol $NAD^+$ under standard GLDH assay conditions, for example at 37 °C and pH 8.0, per one minute.

The inventive liquid reagent contains 0.2 mM to 0.5 mM NADH as a second reagent preferably 0.3 mM to 0.4 mM NADH, and 0.1 mM to 20 mM 2-oxoglutaric acid, preferably 0.5 mM to 10 mM 2-oxoglutaric acid also in the first reagent. These 2 reagents are charged in separated containers so that these are added at the time of reaction.

In accordance with the invention, furthermore, chelating agents are satisfactorily be contained in the first or second liquid reagent. In accordance with the invention, as described above, calcium can be assayed satisfactorily by using the calcium-dependent GLDH. It is firstly confirmed that the enzymological examination of the apparent Km of the glutamate dehydrogenase for calcium is 10 μM and that a linear standard curve within a range of 0 to 4 mM Ca, which is significant for serum calcium assay, can be prepared by adjusting the activity of the glutamate dehydrogenase to enable the determination of serum calcium at a lower level in a linear manner.

Usable chelating agents, include EDTA (ethylenediaminetetraacetic acid), CyDTA (trans-1,2-cyclohexanediamine-N, N, N', N'-tetraacetic acid), DTPA (diethylenetriaminepentaacetic acid), GEDTA (glycol ether diaminetetraacetic acid), TTHA (triethylenetetraminehexaacetic acid), and methyl EDTA (diaminopropane tetraacetic acid) and additionally including NTA (nitrilotriacetic acid), EDTA-OH .(hydroxyethylenediaminetriacetic acid), IDA (iminodiacetic acid), and HIDA (hydroxyethyliminodiacetic acid). NTA is preferably illustrated as one example of the chelating agents.

In accordance with the invention, the inventive assay reagent satisfactorily contains the adjusting factor described above in the first or second reagent. In this case, the content of the adjusting factor is 0.1 to 100 mM; the content of NTA is preferably 1 to 50 mM. Depending on the type of the chelating agents described above, appropriately, the content varies.

(Method for assaying calcium derived from biological material)

Using the inventive reagent, calcium derived from a biological material can be assayed. More specifically, the inventive method uses a single enzyme, namely a specific inactive-type GLDH, which is converted to active-type GLDH, depending on the calcium ion level in the biological material, to assay the enzyme activity of the active-type GLDH to determine the calcium ion level. By concurrently using such chelating agent, calcium at a lower concentration in a sample can also be assayed.

In accordance with the invention, any sample possibly containing such body fluids can be assayed, with no specific limitation, and includes blood, serum, plasma and pancreatic juice. The sample is satisfactorily collected from living organisms.

In accordance with the invention, a specific inactive-type calcium-dependent GLDH is converted to active-type GLDH by using calcium present in a biological material. Subsequently, a reaction solution containing 2-oxoglutaric acid and NADH reacts with the resulting GLDH. The reaction is progressed at pH 6 to 9, preferably at 25 °C to 40 °C for 5 minutes to one hour.

The enzyme activity of the active-type GLDH as converted by the presence of calcium ion can be assayed by known methods. Because NADPH exerts a specific absorption profile at 340 nm, the change of the absorbance at the wave length, as measured prior to and after GLDH reaction, is compared with the data of calcium conversion of GLDH , to determine the calcium level derived from a biological material. The above example is a simple illustration, with no specific limitation.

As has been described above, preferably, a more linear standard curve can be yielded by adding a chelating agent as an adjusting factor to exert its action according to the inventive method (formula 2). In this case, satisfactorily, the chelating agent composing the inventive liquid reagent for calcium assay can be allowed to exert its action prior to or during calcium conversion of inactive-type GLDH to active-type GLDH.

The invention is now described in more detail in the following examples.

Example 1

Calcium-dependent GLDH derived from maize was expressed, extracted and purified as follows.

a. Intrabacterial expression of calcium-dependent GLDH derived from maize

The full-length calcium-dependent GLDH gene from maize is described and reported in the following reference. Sakakibara, H. et al., "Isolation and characterization of a cDNA that encodes maize glutamate dehydrogenase", Plant Cell Physiol. 36, 5: 789-797, 1995.

A 1.5-kb DNA fragment containing the calcium-dependent GLDH gene and ranging from the restriction BspHI site to the restriction EcoRI site in the full-length region was inserted in the multicloning NcoI/EcoRI site of a commercially available expression vector pTrc99A (Pharmacia Co.), to construct a plasmid pTrcZmGLDH expressing maize glutamate dehydrogenase. A transformant was recovered in a host Escherichia coli (abbreviated sometimes as E. coli) strain JM105. The transformant E. coli JM105/pTrcZmGLDH was cultured in an LB culture medium (0.5 % yeast extract, 1 % tryptone, and 1 % sodium chloride, pH 7.5) containing 50 μg/ml sodium ampicillin, and when the transformant reached its logarithmic growth phase, an expression inducer of recombinant glutamate dehydrogenase, namely 1 mM IPTG (isopropylthio-β-galactoside), was added to the culture. The transformant was recovered at a wet weight yield of 6 g, by shaking culture in a flask containing the culture broth of 2 liters. Glutamate dehydrogenase was expressed and accumulated at the total activity of 2, 348 units in the bacteria. Herein, the enzyme activity of the glutamate dehydrogenase was assayed under the following conditions according to the aforementioned formula 2.

As illustrated above, in accordance with the invention, GLDH can efficiently be produced by using the transformant Escherichia coli strain prepared by inserting a recombinant plasmid containing at least a part of the gene encoding GLDH in the host strain. The transformant is designated Escherichia coli JM105/pTrcZmGLDH and is deposited as Accession No. FERM BP-6705 under the Budapest Treaty in National Institute of Bioscience and Human-Technology, Agency of Industrial Science and Technology, of 1-3, Higashi 1 chome, Tsukuba-shi, Ibarakiken, Japan (the deposit date: April 19, 1999).

(Method for assaying thermo-resistant GLDH)

Assay temperature: 37 °C
Assay wave length: 340 nm
Reaction solution pH: 8.0

Substrate mixture solution:

100 mM TEA-HCl, pH 8.0
1 mM $CaCl_2$
5 mM 2-oxoglutaric acid
0.2 M $NH_4Cl$
0.3 mM NADH

One unit of the'activity can generate 1 μmol $NAD^+$ per one minute.

b.      Extraction and purification of maize-derived recombinant GLDH

The starting material transformant of 6 g (in wet weight) recovered through the flask culture was subjected to disruption and extraction of the bacteria and thermal treatment thereof and a combination of various chromatographic means, to elevate the purification ratio of the extracted solution of the disrupted bacteria to 165 fold. The specific activity of the purified enzyme sample was 660 U/mg. The purification process is schematically shown in the following Table 1.

Table 1

| Purification process | Protein (mg) | Total activity (U) | Specific activity (U/mg) | Purification ratio |
|---|---|---|---|---|
| Ultrasonic disruption/enzyme extraction | 590 | 2348 | 4.0 | 1 |
| Supernatant after thermal treatment at 70 °C | 472 | 2111 | 4.5 | 1.1 |
| Ion exchange chromatography on DEAE-Toyo pearl column | 10.6 | 1609 | 152 | 38 |
| Hydrophobic chromatography on phenyl-Toyo pearl column | 1.8 | 1200 | 660 | 165 |
| Note: Protein content was calculated at $E_{280nm}^{1\%}$ = 10.0. | | | | |

c.   Maize-derived recombinant GLDH activity

The enzyme activity of the maize-derived calcium-dependent GLDH recovered by the extraction and purification process was assayed under the conditions described in Example 1a. Consequently, purified maize-derived GLDH was recovered at a yield of 40, 000 units from 40 liters of the bacterial culture. The resulting calcium-dependent GLDH exerted the following properties.

Molecular weight: 290 kDa (6-mer) by gel filtration
Subunit molecular weight: 44, 091
pH activity: optimum pH 7.0 to 8.0
pH stability: pH 5 to 11 (20-min treatment at 70 °C)
Metal ion activation: Km at about 10 μM for $Ca^{2+}$ ($Ca^{++}$); other metal ions never work to activate the enzyme.
Action temperature: 20 °C to 80 °C

Thermal stability: retaining 80 % or more of the initial activity after thermal treatment at 70 °C (at pH 7.0 for 20 minutes)
Stability in solution under storage: retaining 60 % or more of the initial activity after one-week accelerated tests at 37 °C despite the addition or no addition of an activating agent calcium ion.

Example 2

Adjustment of enzyme activity for calcium assay

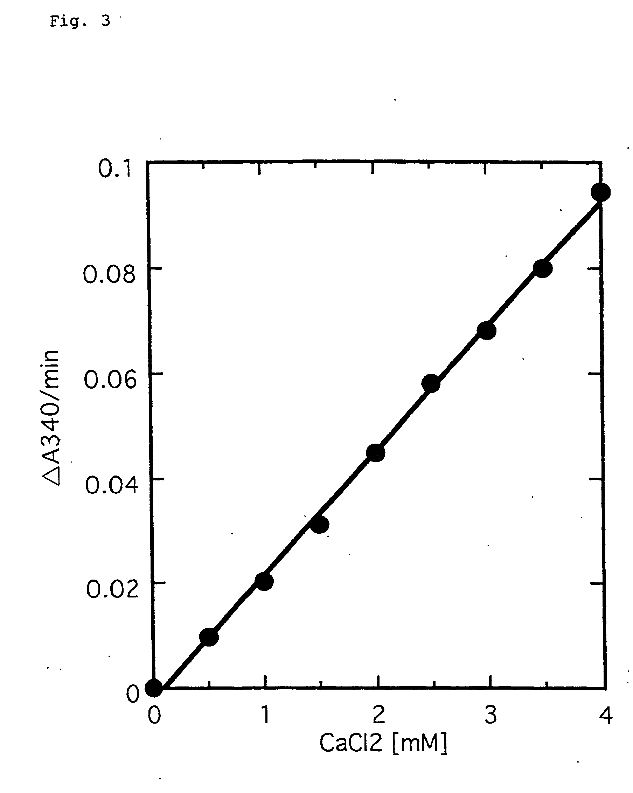

Using the calcium-dependent GLDH recovered in Example 1, assessment was made about the adjustment of the enzyme activity for calcium concentration for assaying serum calcium level. So as to examine the activation in response to the concentration within a range of serum calcium concentration, the activation of maize-derived GLDH by calcium concentration in the presence of various chelating agents added as adjusting factors, each at 0.2 mM, was analyzed in a pattern. On the axis of abscissa is shown the sample calcium concentration; on the axis of ordinate is shown the relative ratio (%) of enzyme activation. The results are shown in Fig. 3. It is indicated that the addition of various chelating agents can adjust the calcium activation. At a 0-4 mM calcium concentration in a sample, NTA is particularly effective as an adjusting factor to permit almost linear activation response.

(Preparation of reaction test solution)

First reagent:

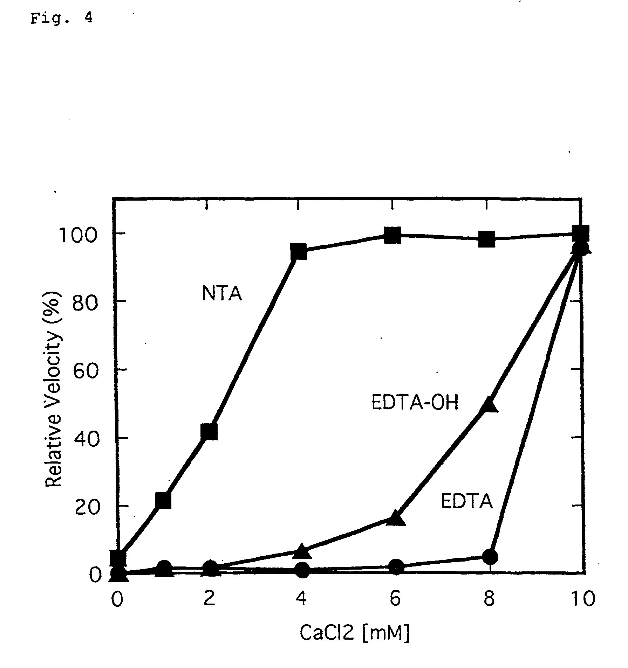

100 mM TEA-HCl buffer,' pH 7.5
0.2 M $NH_4Cl$
10 mM 2-oxoglutaric acid
0.1 U/ml maize-derived GLDH
0.2 mM each of various chelating agents in Fig. 4.

Second reagent:

100 mM TEA-HCl buffer, pH 7.5
0.3 mM NADH

(Calcium sample solution)

Assay apparatus: Automatic analyzer Cobas Fara (manufactured by Roche, Co.)
Assay temperature: 37 °C
Assay wave length: 340 nm

Ratio of added reagents:

sample : pure water : first reagent : second reagent = 6:4:240:60 (µl)

Calculation of activation ratio:

Second reagent containing NADH is added to a mixture of a calcium sample solution and the first reagent, to determine the calcium activation pattern based on the initial velocity of enzyme reaction (triangle A340/min).

Example 3

Assay of serum calcium

Based on the results of Example 2, furthermore, the relation between the NTA concentration and the calcium standard curve was examined. It was found that a linear standard curve could be prepared by using an assay reagent prepared as described below. The results are shown in Fig. 4. Additionally, the calcium level in a commercially available serum (Consera as Sample 1; manufactured by Nissui Pharmaceuticals, Co.) was assayed by using a standard curve prepared by using an aqueous calcium chloride solution as the standard solution; the recovery rate of calcium was calculated by using Sample 2 prepared by adding 1 mM calcium chloride to Sample 1. Consequently, as shown in the following Table 2, the recovery rate of calcium added to the Sample 1 from the Sample 2 was calculated to be 100.2 %. It was indicated that the serum calcium was specifically assayed by using the prepared reagent.

Table 2

| Sample | Assayed calcium (mmol/liter) | CV (n = 10) |
|---|---|---|
| Sample 1 (human serum) | 1.87 | 1.7 % |
| Sample 2 (Sample 1 + 1 mM CaCl$_2$) | 2.89 | 2.2 % |

(Preparation of serum calcium assay reagent)

First reagent:

100 mM TEA-HCl buffer, pH 7.5
10 mM 2-oxoglutaric acid
200 mM NH$_4$Cl
0.3 U/ml maize-derived GLDH

Second reagent:

10 mM NaHCO$_3$-NaOH buffer, pH 9.0
1.2 mM NADH
20 mM NTA

(Calculation of absorbance change for assaving serum calcium)

Calcium was assayed by the procedures described in Example 2.

Advantages of the Invention

As has been described above, the inventive liquid reagent can retain its high enzyme activity in solution for a long term because of the use of a specific calcium-dependent GLDH therein. The reagent is very stable in a solution state under storage, so the reagent can be incorporated in a liquid reagent kit with ready handling, to enable simple and accurate calcium assay in body fluid samples.

**Claims**

1. A liquid reagent for the use for assaying calcium in body fluid same, which consists of a kit containing two separate containers containing a first reagent containing 2-oxoglutaric acid and calcium-dependent glutamate dehydrogenase, and a second reagent containing NADH; wherein the first reagent or the second reagent contains, further, one or more chelating agents(s) for calcium, and wherein the calcium-dependent glutamate dehydrogenase is one obtained from maize or one produced by using a microbial transformant containing an expression plasmid capable of expressing the same calcium-dependent glutamate dehydrogenase as said one obtained from maize, wherein the calcium-dependent glutamate dehydrogenase is of an amino acid sequence of SEQ ID No.1.

2. The liquid reagent according to claim 1, for the use for assaying calcium in blood serum.

3. The liquid reagent according to any one of claims 1 and 2, wherein said one or more chelating agent(s) is (are) selected from the group consisting of ethylenediaminetetraacetic acid, trans-1,2-cyclohexanediamine-N,N,N',N'-tetraacetic acid, diethylenetri-aminepentaacetic acid, glycol ether diaminetetraacetic acid, triethylenetetraminehexaacetic acid, diaminopropane tetraacetic acid, nitrilotriacetic acid, hydroxyethylenediaminetriacetic acid, iminodiacetic acid, and hydroxyethyliminodiacetic acid.

4. The liquid reagent according to any one of claims 1 to 3, wherein said microbial transformant is Escherichia coli FERM BP-6705.

5. A method for assaying calcium in body fluid samples which comprises using the liquid reagent according to any one of claims 1 to 5

**Patentansprüche**

1. Flüssiges Reagenz zur Verwendung zur Bestimmung von Calcium in Körperflüssigkeitsproben, welches aus einem Kit besteht, das zwei separate Behälter enthält, enthaltend ein erstes Reagenz, welches 2-Oxoglutarsäure und Calcium-abhängige Glutamat-Dehydrogenase enthält, und ein zweites Reagenz, enthaltend NADH; wobei das erste Reagenz oder das zweite Reagenz darüber hinaus ein oder mehr Chelatisierungsmittel für Calcium enthält, und wobei die Calcium-abhängige Glutamat-Dehydrogenase eine ist, die aus Mais erhalten wird, oder eine, die durch Verwendung einer mikrobiellen Transformante hergestellt wird, welche ein Expressionsplasmid enthält, das dieselbe Calcium-abhängige Glutamat-Dehydrogenase wie die aus Mais erhaltene exprimieren kann, wobei die Calcium-abhängige Glutamat-Dehydrogenase eine Aminosäuresequenz mit SEQ ID No. 1 aufweist.

2. Flüssiges Reagenz nach Anspruch 1 zur Verwendung zur Bestimmung von Calcium in Blutserum.

3. Flüssiges Reagenz nach einem der Ansprüche 1 und 2, wobei das eine oder mehr Chelatisierungsmittel ausgewählt wird/werden aus der Gruppe, die aus Ethylendiamintetraessigsäure, Trans-1,2-cyclohexandiamin-N,N,N', N'-tetraessigsäure, Diethylentriaminopentaessigsäure, Glykoletherdiamintetraessigsäure, Triethylentetraminhexaessigsäure, Diaminopropantetraessigsäure, Nitrilotriessigsäure, Hydroxyethylendiamintriessigsäure, Iminodiessigsäure und Hydroxyethyliminodiessigsäure besteht.

4. Flüssiges Reagenz nach einem der Ansprüche 1 bis 3, wobei die mikrobielle Transformante Escherichia coli FERM BP-6705 ist.

5. Verfahren zur Bestimmung von Calcium in Körperflüssigkeitsproben, welches Verwendung des flüssigen Reagenz nach einem der Ansprüche 1 bis 5 umfasst.

**Revendications**

1. Réactif liquide à utiliser afin d'évaluer la présence de calcium dans des prélèvements de fluides corporels et qui est constitué d'un kit contenant deux récipients séparés contenant un premier réactif contenant de l'acide 2-oxoglutarique et de la déshydrogénase de glutamate dépendante du calcium et un deuxième réactif contenant de la NADH ; dans lequel le premier réactif ou le deuxième réactif contient, en outre, un ou plusieurs agents chélateurs du calcium et dans lequel la déshydrogénase de glutamate dépendante du calcium est une déshydrogénase obtenue à partir du maïs ou une déshydrogénase produite en utilisant un transformant microbien contenant un plasmide d'expression capable d'exprimer la même déshydrogénase de glutamate dépendante du calcium que ladite déshydrogénase obtenue à partir du maïs, dans lequel la déshydrogénase de glutamate dépendante du calcium a la séquence en acides aminés de No. ID. de SEQ. 1.

2. Réactif liquide selon la revendication 1 à utiliser afin d'évaluer la présence de calcium dans le sérum sanguin.

3. Réactif liquide selon l'une quelconque des revendications 1 et 2 dans lequel ledit ou lesdits un ou plusieurs agents chélateurs sont sélectionnés parmi le groupe constitué de l'acide éthylènediamine-tétracétique, l'acide trans-1,2-cyclohexanediamine-N,N,N',N'-tétracétique, l'acide diéthylènetriamine-pentacétique, l'acide diaminetétracétique d'éther de glycol, l'acide triéthylènetétraminehexacétique, l'acide tétracétique de diaminopropane, l'acide nitrilotriacétique, l'acide hydroxyéthylènediaminetriacétique, l'acide iminodiacétique et l'acide hydroxyéthyliminodiacétique.

4. Réactif liquide selon l'une quelconque des revendications 1 à 3 dans lequel ledit transformant microbien est Escherichia coli FERM BP-6705.

5. Méthode d'évaluation de la présence de calcium dans des prélèvements de fluides corporels qui comprend l'utilisation du réactif liquide selon l'une quelconque des revendications 1 à 5.

Fig. 1

$$2\text{-oxoglutaric acid} + NH_3 + NAD(P)H$$

$$\downarrow\uparrow \qquad\qquad (1)$$

$$\text{glutamic acid} + H_2O + NAD(P)^+$$

Fig. 2

inactive-type GLDH

$$\downarrow \qquad \text{calcium ion}$$

$$(+ \text{adjusting factor})$$

active-type GLDH

$$\downarrow$$

$$\begin{array}{ccc}
\text{2-oxoglutaric acid} & \longrightarrow & \text{glutamic acid} + H_2O \\
+ NH_3 + NAD(P)H & \longleftarrow & + NAD(P)^+
\end{array}$$

$$(2)$$

Fig. 3

Fig. 4